# EUROPÄISCHE PATENTSCHRIFT

(11) **EP 1 680 393 B1**
(45) Veröffentlichungstag und Bekanntmachung des Hinweises auf die Patenterteilung: **29.09.2010**
(21) Anmeldenummer: 04765964.4
(22) Anmeldetag: 15.10.2004
(51) Int. Cl.: C07C 209/84, C07C 209/86, B01D 3/32, B01J 19/32, C07D 295/02

(54) **VERFAHREN ZUR DESTILLATIVEN AUFTRENNUNG VON GEMISCHEN ENTHALTEND ETHYLENAMINE**
METHOD FOR THE DISTILLATIVE SEPARATION OF MIXTURES CONTAINING ETHYLENEAMINES
PROCEDE DE FRACTIONNEMENT PAR DISTILLATION DE MELANGES CONTENANT DES ETHYLENAMINES

(30) Priorität: 17.10.2003 DE 10349059
(43) Veröffentlichungstag der Anmeldung: 19.07.2006
(73) Patentinhaber: BASF SE, 67056 Ludwigshafen (DE)
(72) Erfinder: SIEGERT, Markus, 69126 Heidelberg (DE); MELDER, Johann-Peter, 67459 Böhl-Iggelheim (DE); KRUG, Thomas, 67550 Worms (DE); NOUWEN, Jan, B-2960 Brecht (BE)
(86) Internationale Anmeldenummer: PCT/EP2004/011584
(87) Internationale Veröffentlichungsnummer: WO 2005/037769

(56) Entgegenhaltungen:
- DE-A1- 19 933 850
- US-A1- 2004 220 406
- KAIBEL G: "DISTILLATION COLUMNS WITH VERTICAL PARTITIONS" CHEMICAL ENGINEERING AND TECHNOLOGY, WEINHEIM, DE, Bd. 10, 1987, Seiten 92-98, XP002939161 ISSN: 0930-7516
- LESTAK ET AL: "ADVANCED DISTILLATION SAVESE" CHEMICAL ENGINEERING, MCGRAW-HILL, ALBANY, NY, US, Bd. 7, Juli 1997 (1997-07), Seiten 72-76, XP001156299 ISSN: 0009-2460
- SCHULTZ M A ET AL: "REDUCE COSTS WITH DIVIDING-WALL COLUMNS" CHEMICAL ENGINEERING PROGRESS, AMERICAN INSTITUTE OF CHEMICAL ENGINEERS. NEW YORK, US, Bd. 98, Nr. 5, Mai 2002 (2002-05), Seiten 64-71, XP001106017 ISSN: 0360-7275

## Beschreibung

Verfahren zur destillativen Auftrennung von Gemischen enthalten Ethylenamine gemäß Anspruch 1.

Die vorliegende Erfindung betrifft ein Verfahren zur destillativen Auftrennung von Gemischen enthaltend Ethylenamine, gemäß Anspruch 1.

Für die destillative, z.B. kontinuierliche Zerlegung von Mehrstoffgemischen sind verschiedene Verfahrensvarianten gebräuchlich. Im einfachsten Fall wird das aufzutrennende Gemisch (Zulaufgemisch) in zwei Fraktionen, eine leichtsiedende Kopffraktion und eine schwersiedende Sumpffraktion, zerlegt.

Bei der Auftrennung von Zulaufgemischen in mehr als zwei Fraktionen müssen nach dieser Verfahrensvariante mehrere Destillationskolonnen eingesetzt werden. Um den apparativen Aufwand zu begrenzen, setzt man bei der Auftrennung von Vielstoffgemischen nach Möglichkeit Kolonnen mit flüssigen oder dampfförmigen Seitenabzügen ein.

Die Anwendungsmöglichkeit von Destillationskolonnen mit Seitenabzügen ist jedoch dadurch stark eingeschränkt, dass die an den Seitenabzugsstellen entnommenen Produkte selten oder nie völlig rein sind. Bei Seitenentnahmen im Verstärkungsteil der Kolonne, die üblicherweise in flüssiger Form erfolgen, enthält das Seitenprodukt noch Anteile an leichtsiedenden Komponenten, die über Kopf abgetrennt werden sollen. Entsprechendes gilt für Seitenentnahmen im Abtriebsteil der Kolonne, die meist dampfförmig erfolgen, bei denen das Seitenprodukt noch Hochsiederanteile aufweist.

Die Verwendung von konventionellen Seitenabzugskolonnen ist daher auf Fälle begrenzt, in denen verunreinigte Seitenprodukte zulässig sind.

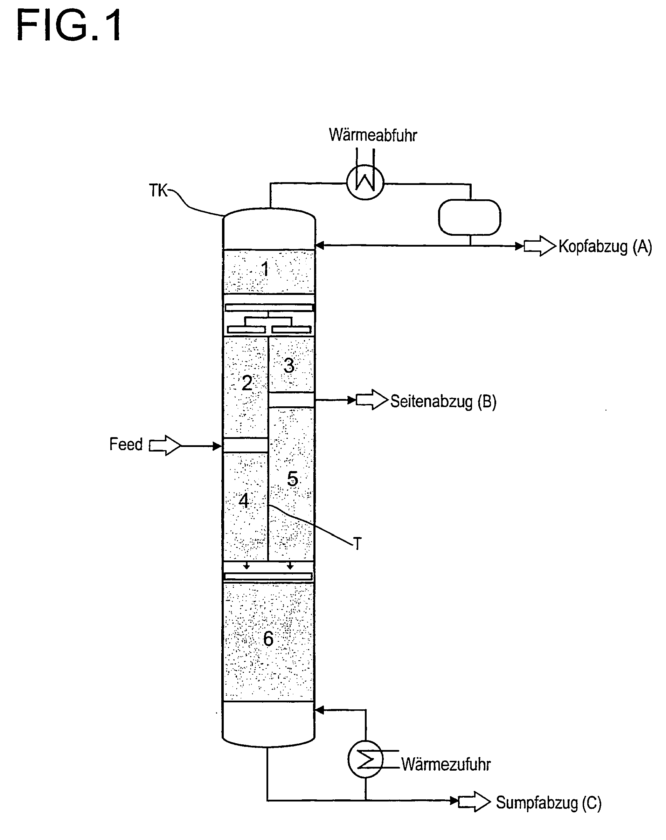

Eine Abhilfemöglichkeit bieten Trennwandkolonnen (siehe z.B. Abbildung 1). Dieser Kolonnentyp ist beispielsweise beschrieben in:
US 2,471,134, US 4,230, 533, EP-A-122 367, EP-A-126 288, EP-A-133 510,
Chem.-Ing.-Tech. 61, (1989), Nr. 1, Seiten 16-25,
Gas Separation and Purification 4 (1990), Seiten 109 - 114,
Process Engineering 2 (1993), Seiten 33 - 34,
Trans IChemE 72 (1994), Part A, Seiten 639 - 644, und

Die Zeitschriftenartikel
Kaibel G.: "Distillation Columns with Vertical Partitions", Chem. Eng. Technol. 10 (1987), Seiten 92 - 98,
Lestak F. und Collins C.: "Advanced Distillation saves Energy & Capital", Chemical Engineering 7 (1997), Seiten 72 - 76, sowie
Schultz M.A. et al: "Reduce Costs with Dividing-wall Columns", Chem. Engng. Progress 98(5) (2002), Seiten 64 - 71
beleuchten unterschiedliche Aspekte des Einsatzes von Trennwandkolonnen zur destillativen Auftrennung von Mehrkomponentengemischen und setzen sich mit Vor- und Nachteilen dieser Technologie im Vergleich zur Trennung in herkömmlichen Kolonnensequenzen auseinander.

Bei dieser Bauart ist es möglich, Seitenprodukte ebenfalls in reiner Form zu entnehmen. Im mittleren Bereich oberhalb und unterhalb der Zulaufstelle und der Seitenentnahme ist eine Trennwand angebracht, die den Zulaufteil gegenüber dem Entnahmeteil abdichtet und in diesem Kolonnenteil eine Quervermischung von Flüssigkeits- und Brüdenströmen unterbindet. Hierdurch verringert sich bei der Auftrennung von Vielstoffgemischen die Zahl der insgesamt benötigten Destillationskolonnen.

Da dieser Kolonnentyp eine apparative Vereinfachung von thermisch gekoppelten Destillationskolonnen darstellt, weist er darüber hinaus auch einen besonders niedrigen Energieverbrauch auf. Eine Beschreibung von thermisch gekoppelten Destillationskolonnen, die in verschiedener apparativer Gestaltung ausgeführt sein können, findet sich ebenfalls in den oben genannten Stellen in der Fachliteratur.

Trennwandkolonnen und thermisch gekoppelte Destillationskolonnen bieten gegenüber der Anordnung von konventionellen Destillationskolonnen sowohl hinsichtlich des Energiebedarfs als auch der Investitionskosten Vorteile und werden daher zunehmend industriell eingesetzt.

Für die Regelung von Trennwandkolonnen und thermisch gekoppelten Kolonnen werden verschiedene Regelungsstrategien beschrieben. Beschreibungen finden sich in:
US 4,230,533, DE-C2-35 22 234, EP-A-780 147,
Process Engineering 2 (1993), 33 - 34, und
Ind. Eng. Chem. Res. 34 (1995), 2094 - 2103.

Die ältere deutsche Patentanmeldung Nr. 10335991.5 vom 01.08.03 betrifft ein Verfahren zur Herstellung von Ethylenaminen durch Umsetzung von Monoethanolamin (MEOA) mit Ammoniak in Gegenwart eines Katalysators und Auftrennung des resultierenden Reaktionsaustrags in Destillationskolonnen.

Die deutsche Patentanmeldung DE 199 33 850 A1 betrifft ein Verfahren zur Reinigung von Triethylendiamin (TEDA) durch fraktionierende Destillation, wobei man das bei der Destillation anfallende gasförmige TEDA in ein flüssiges Lösungsmittel einleitet.

Der vorliegenden Erfindung lag die Aufgabe zugrunde, ein verbessertes wirtschaftliches Verfahren zur Auftrennung von Gemischen enthaltend Ethylenamine aufzufinden. Die einzelnen Ethylenamine, insbesondere Ethylendiamin (EDA), Piperazin (PIP), Diethylentriamin (DETA) und Aminoethylethanolamin (AEEA), sollten dabei in hoher Reinheit und Qualität (z.B. Farbqualität) anfallen.

Demgemäß wurde ein Verfahren zur destillativen Auftrennung von Gemischen enthaltend Ethylenamine gefunden, welches dadurch gekennzeichnet ist, dass es sich bei dem Gemisch enthaltend Ethylenamine um ein Produkt, erhalten durch Umsetzung von Monoethanolamin (MEOA) mit Ammoniak und nachfolgender teilweiser oder vollständiger Abtrennung von Ammoniak und Wasser, handelt, es sich bei den Ethylenaminen um Ethylendiamin (EDA), Piperazin (PIP), Diehtylentriamin (DETA), Aminoethylethanolamin (AEEA) und/oder Monoethanolamin (MEOA) handelt und die Auftrennung in einer oder mehreren Trennwandkolonnen durchgeführt wird.

Z.B. können EDA, DETA, PIP und AEEA mit den in US 2,861,995 (Dow), DE-A-1 172 268 (BASF) und US 3,112,318 (Union Carbide), (vgl. Ullmann's Encyclopedia of Industrial Chemistry, 6th Edition, 2000 Electronic Release, Chapter 8.1.1: 1,2-Diaminoethane), beschriebenen Verfahren aus MEOA und Ammoniak hergestellt werden, wobei Ammoniak z.B. im ein- bis zwanzigfachen molaren Überschuss eingesetzt wird und MEOA zu z.B. 40 bis 60 % umgesetzt wird. Das Austragsgemisch dieser Umsetzungen, bestehend überwiegend aus Ammoniak, Wasser, MEOA, EDA, DETA, PIP, AEEA und höher siedenden Ethylenaminen und Ethylenaminoalkoholen, wird zunächst entspannt und ausgegast und anschließend werden Ammoniak und Wasser destillativ abgetrennt. Das erfindungsgemäße Verfahren eignet sich insbesondere zur weiteren kontinuierlichen Aufarbeitung des nach der Entwässerung verbleibenden Gemisches aus EDA, PIP, (unumgesetzten) MEOA, DETA, AEEA und weiteren höhersiedenden Nebenprodukten.

Eine typische, im erfindungsgemäßen Verfahren anzuwendende Trennwandkolonne (TK) (siehe Abbildung 1) weist jeweils eine Trennwand (T) in Kolonnenlängsrichtung unter Ausbildung eines oberen gemeinsamen Kolonnenbereichs (1), einen unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (3) und Abtriebsteil (5) auf, wobei die Zuführung des aufzutrennenden Gemischs (Feed) im mittleren Bereich des Zulaufteils (2, 4), die Abführung der Hochsiederfraktion über Sumpf (Sumpfabzug C), die Abführung der Leichtsiederfraktion über Kopf (Kopfabzug A) und die Abführung der Mittelsiederfraktion aus dem mittleren Bereich des Entnahmeteils (3, 5) (Seitenabzug B) erfolgt.

Die Trennwandkolonne/n des erfindungsgemäßen Verfahrens weist / weisen jeweils bevorzugt 30 bis 100, insbesondere 50 bis 90, theoretische Trennstufen auf.

Das Gemisch enthaltend Ethylenamine wird bevorzugt in einer Trennwandkolonne aufgearbeitet, in der EDA, insbesondere EDA mit einer Reinheit > 99,0 Gew.-%, als Kopfprodukt und PIP, insbesondere PIP mit einer Reinheit > 99,0 Gew.-%, als Seitenabzugsstrom gewonnen wird, wobei der Betriebsdruck im Allgemeinen bei 0,1 bis 5 bar, bevorzugt bei 0,3 bis 2 bar, besonders bevorzugt 0,7 bis 1,6 bar, beträgt.

Unter ,Betriebsdruck' ist in diesem Dokument der am Kopf der Kolonne gemessene absolute Druck zu verstehen.

Bevorzugt wird nach der Abtrennung von EDA und PIP in einer Trennwandkolonne weiter aufgearbeitet, in der MEOA als Kopfprodukt gewonnen wird und DETA, insbesondere DETA mit einer Reinheit > 99,0 Gew.-%, als Seitenabzugsstrom gewonnen wird, wobei der Betriebsdruck im Allgemeinen bei 0,01 bis 2,5 bar, bevorzugt 0,01 bis 0,70 bar, insbesondere 0,05 bis 0,25 bar, beträgt.

Nach der Abtrennung von EDA, PIP, MEOA und DETA wird bevorzugt in einer Trennwandkolonne weiter aufgearbeitet, in der AEEA, insbesondere AEEA mit einer Reinheit > 99,0 Gew.-%, als Seitenabzugsstrom gewonnen wird, wobei der Betriebsdruck im Allgemeinen bei 0,001 bis 1,0 bar, bevorzugt 0,001 bis 0,05 bar, insbesondere 0,005 bis 0,025 bar, beträgt.

Die Trennwandkolonnen werden insbesondere so verschaltet, dass das Rohgemisch aus der Synthese von Ethylenaminen nach der teilweisen oder vollständigen Abtrennung von Ammoniak und Wasser der ersten Trennwandkolonne zugeführt wird, in der reines EDA als Kopfprodukt und reines PIP als Seitenabzugsstrom gewonnen wird, und dass der Sumpfstrom dieser Kolonne in der zweiten Trennwandkolonne weiter aufgearbeitet wird, in der MEOA als Kopfprodukt und reines DETA als Seitenabzugsstrom gewonnen wird, und der Sumpfstrom der zweiten Trennwandkolonne einer dritten Trennwandkolonne zugeführt wird, in welcher reines AEEA als Seitenabzugsstrom gewonnen wird.

Das Sumpfprodukt der Trennwandkolonne zur Gewinnung von AEEA wird bevorzugt in einer oder mehreren weiteren konventionellen Destillationskolonnen zur Aufkonzentrierung und Reinigung weiterer, höher siedender Ethylenamine und/oder Ethylenaminoalkohole weiter aufgearbeitet.

Höher siedende Ethylenamine und/oder Ethylenaminoalkohole sind hier solche Amine, die (bei gleichem Druck) einen höheren Siedepunkt als AEEA aufweisen.

In einer alternativen Verfahrensweise wird der Sumpfstrom der oben aufgeführten Trennwandkolonne zur Abtrennung von EDA und PIP in weiteren konventionellen Destillationskolonnen weiter aufgearbeitet, wobei zuerst MEOA in einer Destillationskolonne als Kopfprodukt gewonnen wird und aus dem Sumpfstrom dieser Kolonne in der nächsten Kolonne DETA, insbesondere DETA mit einer Reinheit > 99,0 Gew.-%, als Kopfprodukt gewonnen wird, und der Sumpfstrom dieser Kolonne
(a) einer oder mehreren weiteren konventionellen Kolonnen zugeführt wird, um AEEA, insbesondere AEEA mit einer Reinheit > 99,0 Gew.-%, zu gewinnen, oder
(b) einer Trennwandkolonne zugeführt wird, in der AEEA, insbesondere AEEA mit einer Reinheit > 99,0 Gew.-%, als Seitenabzugsstrom gewonnen wird.

In einer weiteren alternativen Verfahrensweise wird das Gemisch enthaltend Ethylenamine einer konventionellen Destillationskolonne zugeführt, in der ein EDA/PIP-Gemisch als Kopfprodukt gewonnen wird, und in einer weiteren konventionellen Kolonne in EDA, insbesondere EDA mit einer Reinheit > 99,0 Gew.-%, und PIP, insbesondere PIP mit einer Reinheit > 99,0 Gew.-%, aufgetrennt, und der Sumpfstrom dieser Kolonne in einer Trennwandkolonne weiter aufgearbeitet, so dass MEOA als Kopfprodukt gewonnen wird und DETA, insbesondere DETA mit einer Reinheit > 99,0 Gew.-%, als Seitenabzugsstrom anfällt, und der Sumpfstrom dieser Trennwandkolonne
(a) einer oder mehreren konventionellen Destillationskolonnen zugeführt wird, um AEEA, insbesondere AEEA mit einer Reinheit > 99,0 Gew.-%, zu gewinnen, oder
(b) einer weiteren Trennwandkolonne zugeführt wird, in der AEEA, insbesondere AEEA mit einer Reinheit > 99,0 Gew.-%, als Seitenabzugsstrom gewonnen wird.

Insbesondere weist im erfindungsgemäßen Verfahren der obere gemeinsame Kolonnenbereich (1) der Trennwandkolonne (TK) zur Abtrennung von EDA und PIP 5 bis 50 %, bevorzugt 20 bis 35 %, der Verstärkungsteil (2) des Zulaufteils (2, 4) der Kolonne 5 bis 50 %, bevorzugt 10 bis 20 %, der Abtriebsteil (4) des Zulaufteils der Kolonne 5 bis 50 %, bevorzugt 20 bis 35 %, der Verstärkungsteil (3) des Entnahmeteils (3, 5) der Kolonne 5 bis 50 %, bevorzugt 7 bis 20 %, der Abtriebsteil (5) des Entnahmeteils der Kolonne 5 bis 50 %, bevorzugt 20 bis 35 %, und der gemeinsame untere Bereich (6) der Kolonne 5 bis 50 %, bevorzugt 20 bis 35 %, der Gesamtzahl der theoretischen Trennstufen der Kolonne auf.

Insbesondere weist im erfindungsgemäßen Verfahren der obere gemeinsame Kolonnenbereich (1) der Trennwandkolonne (TK) zur Abtrennung von MEOA und DETA 5 bis 50 %, bevorzugt 5 bis 15 %, der Verstärkungsteil (2) des Zulaufteils (2, 4) der Kolonne 5 bis 50 %, bevorzugt 25 bis 40 %, der Abtriebsteil (4) des Zulaufteils der Kolonne 5 bis 50 %, bevorzugt 20 bis 35 %, der Verstärkungsteil (3) des Entnahmeteils (3, 5) der Kolonne 5 bis 50 %, bevorzugt 15 bis 25 %, der Abtriebsteil (5) des Entnahmeteils der Kolonne 5 bis 50 %, bevorzugt 40 bis 55 %, und der gemeinsame untere Bereich (6) der Kolonne 5 bis 50 %, bevorzugt 15 bis 25 %, der Gesamtzahl der theoretischen Trennstufen der Kolonne auf.

Insbesondere weist im erfindungsgemäßen Verfahren der obere gemeinsame Kolonnenbereich (1) der Trennwandkolonne (TK) zur Abtrennung von AEEA 5 bis 50 %, bevorzugt 5 bis 30 %, der Verstärkungsteil (2) des Zulaufteils (2, 4) der Kolonne 5 bis 50 %, bevorzugt 15 bis 35 %, der Abtriebsteil (4) des Zulaufteils der Kolonne 5 bis 50 %, bevorzugt 15 bis 35 %, der Verstärkungsteil (3) des Entnahmeteils (3, 5) der Kolonne 5 bis 50 %, bevorzugt 15 bis 35 %, der Abtriebsteil (5) des Entnahmeteils der Kolonne 5 bis 50 %, bevorzugt 15 bis 35 %, und der gemeinsame untere Bereich (6) der Kolonne 5 bis 50 %, bevorzugt 10 bis 25 %, der Gesamtzahl der theoretischen Trennstufen der Kolonne auf.

Insbesondere beträgt in der Trennwandkolonne (TK) die Summe der Zahl der theoretischen Trennstufen der Teilbereiche (2) und (4) im Zulaufteil 80 bis 110 %, bevorzugt 90 bis 100 %, der Summe der Zahl der Trennstufen der Teilbereiche (3) und (5) im Entnahmeteil.

Das erfindungsgemäße Verfahren ist bevorzugt **dadurch gekennzeichnet, dass** die Zulaufstelle und die Seitenabzugsstelle der Trennwandkolonne zur Abtrennung von EDA und PIP hinsichtlich der Lage der theoretischen Trennstufen auf unterschiedlicher Höhe in der Kolonne angeordnet sind, indem sich die Zulaufstelle um 1 bis 10, insbesondere 1 bis 5, theoretische Trennstufen von der Seitenabzugsstelle unterscheidet.

Das erfindungsgemäße Verfahren ist bevorzugt **dadurch gekennzeichnet, dass** die Zulaufstelle und die Seitenabzugsstelle der Trennwandkolonne zur Abtrennung von MEOA und DETA hinsichtlich der Lage der theoretischen Trennstufen auf unterschiedlicher Höhe in der Kolonne angeordnet sind, indem sich die Zulaufstelle um 1 bis 20, insbesondere 5 bis 15, theoretische Trennstufen von der Seitenabzugsstelle unterscheidet.

Das erfindungsgemäße Verfahren ist bevorzugt **dadurch gekennzeichnet, dass** die Zulaufstelle und die Seitenabzugsstelle der Trennwandkolonne zur Abtrennung von AEEA hinsichtlich der Lage der theoretischen Trennstufen auf unterschiedlicher Höhe in der Kolonne angeordnet sind, indem sich die Zulaufstelle um 1 bis 20, insbesondere 5 bis 15, theoretische Trennstufen von der Seitenabzugsstelle unterscheidet.

Falls besonders hohe Anforderungen an die Reinheiten der Produkte gestellt werden, ist es günstig, die Trennwand mit einer thermischen Isolierung auszustatten. Eine Beschreibung der verschiedenen Möglichkeiten der thermischen Isolierung der Trennwand findet sich in EP-A-640 367. Eine doppelwandige Ausführung mit einem zwischenliegenden engen Gasraum ist besonders günstig.

Bevorzugt ist der durch die Trennwand (T) unterteilte Teilbereich der Kolonne (TK) bestehend aus den Teilbereichen 2, 3, 4 und 5 oder Teilen davon mit geordneten Packungen oder Füllkörpern bestückt und die Trennwand in diesen Teilbereichen wärmeisolierend ausgeführt.

Bevorzugt ist alternativ der durch die Trennwand (T) unterteilte Teilbereich der Kolonne (TK) bestehend aus den Teilbereichen 2, 3, 4 und 5 oder Teilen davon mit Böden bestückt und die Trennwand in diesen Teilbereichen wärmeisolierend ausgeführt.

Im erfindungsgemäßen Verfahren wird die Mittelsiederfraktion an der Seitenabzugsstelle in flüssiger Form oder gasförmig entnommen.

Bevorzugt wird der Brüdenstrom am unteren Ende der Trennwand (T) durch die Wahl und/oder Dimensionierung der Trenneinbauten und/oder den Einbau druckverlusterzeugender Vorrichtungen, beispielsweise von Blenden, so eingestellt, dass das Verhältnis des Brüdenstroms im Zulaufteil zu dem des Entnahmeteils 0,8 bis 1,2, insbesondere 0,9 bis 1,1, beträgt.

Die in diesem Dokument genannten Verhältnisse bezüglich bestimmter Ströme (z.B. Flüssigkeitsströme, Brüdenströme, Sumpfströme, Zulaufströme, Seitenabzugsströme) beziehen sich auf das Gewicht.

Bevorzugt wird die aus dem oberen gemeinsamen Bereich (1) der Kolonne ablaufende Flüssigkeit in einem in der Kolonne oder außerhalb der Kolonne angeordneten Auffangraum gesammelt und gezielt durch eine Festeinstellung oder Regelung am oberen Ende der Trennwand (T) so aufgeteilt, dass das Verhältnis des Flüssigkeitsstroms zum Zulaufteil zu dem zum Entnahmeteil 0,1 bis 1,0, insbesondere 0,25 bis 0,8, beträgt.

Im erfindungsgemäßen Verfahren wird bevorzugt die Flüssigkeit auf den Zulaufteil (Feed) über eine Pumpe gefördert oder über eine statische Zulaufhöhe von mindestens 1 m mengengeregelt aufgegeben und die Regelung so eingestellt, dass die auf den Zulaufteil aufgegebene Flüssigkeitsmenge nicht unter 30 % des Normalwertes sinken kann.

Im erfindungsgemäßen Verfahren wird bevorzugt die Aufteilung der aus dem Teilbereich 3 im Entnahmeteil der Kolonne ablaufenden Flüssigkeit auf den Seitenabzug und auf den Teilbereich 5 im Entnahmeteil der Kolonne durch eine Regelung so eingestellt, dass die auf den Teilbereich 5 aufgegebene Flüssigkeitsmenge nicht unter 30 % des Normalwertes sinken kann.

Bevorzugt ist weiterhin, dass die Trennwandkolonne (TK) am oberen und unteren Ende der Trennwand (T) Probenahmemöglichkeiten aufweist und aus der Kolonne kontinuierlich oder in zeitlichen Abständen flüssig oder gasförmig Proben entnommen und hinsichtlich ihrer Zusammensetzung untersucht werden.

Im erfindungsgemäßen Verfahren wird bevorzugt das Aufteilungsverhältnis der Flüssigkeit am oberen Ende der Trennwand (T) so eingestellt, dass die Konzentration an denjenigen Komponenten der Hochsiederfraktion, für die im Seitenabzug ein bestimmter Grenzwert für die Konzentration erzielt werden soll, in der Flüssigkeit am oberen Ende der Trennwand 5 bis 75 %, insbesondere 5 bis 40 %, des Wertes ausmacht, der im Seitenabzugsprodukt erzielt werden soll, und die Flüssigkeitsaufteilung dahingehend eingestellt, dass bei höheren Gehalten an Komponenten der Hochsiederfraktion mehr und bei niedrigeren Gehalten an Komponenten der Hochsiederfraktion weniger Flüssigkeit auf den Zulaufteil geleitet wird.

Im erfindungsgemäßen Verfahren wird bevorzugt die Heizleistung im Verdampfer so eingestellt, dass die Konzentration an denjenigen Komponenten der Leichtsiederfraktion, für die im Seitenabzug ein bestimmter Grenzwert für die Konzentration erzielt werden soll, am unteren Ende der Trennwand (T) so eingestellt wird, dass die Konzentration an Komponenten der Leichtsiederfraktion in der Flüssigkeit am unteren Ende der Trennwand 10 bis 99 %, bevorzugt 25 bis 97,5 %, des Wertes ausmacht, der im Seitenabzugsprodukt erzielt werden soll, und die Heizleistung dahingehend eingestellt, dass bei höherem Gehalt an Komponenten der Leichtsiederfraktion die Heizleistung erhöht und bei niedrigerem Gehalt an Komponenten der Leichtsiederfraktion die Heizleistung verringert wird.

Das erfindungsgemäße Verfahren ist bevorzugt **dadurch gekennzeichnet, dass** die Destillatentnahme temperaturgeregelt erfolgt und als Regeltemperatur eine Messstelle im Teilbereich 1 der Kolonne verwendet wird, die um 2 bis 20, insbesondere 4 bis 15, theoretische Trennstufen unterhalb des oberen Endes der Kolonne angeordnet ist.

Das erfindungsgemäße Verfahren ist bevorzugt dadurch gekennzeichnet , dass die Entnahme des Sumpfprodukts temperaturgeregelt erfolgt und als Regeltemperatur eine Messstelle im Teilbereich 6 der Kolonne verwendet wird, die um 2 bis 20, insbesondere 4 bis 15, theoretische Trennstufen oberhalb des unteren Endes der Kolonne angeordnet ist.

In einer weiteren besonderen Ausgestaltung erfolgt die Entnahme des Seitenprodukts im Seitenabzug standgeregelt und als Regelgröße wird der Flüssigkeitsstand im Verdampfer verwendet.

Eine weitere erfindungsgemäße Variation des Verfahrens zur destillativen Aufarbeitung von Ethylenaminen besteht darin, dass anstelle einer der genannten Trennwandkolonnen eine Zusammenschaltung von zwei Destillationskolonnen in Form einer thermischen Kopplung verwendet wird.

Bevorzugt sind beide thermisch gekoppelten Destillationskolonnen jeweils mit einem eigenen Verdampfer und Kondensator ausgestattet.

Weiterhin werden bevorzugt die beiden thermisch gekoppelten Kolonnen bei verschiedenen Drücken betrieben und in den Verbindungsströmen zwischen den beiden Kolonnen nur Flüssigkeiten gefördert.

Bevorzugt bei der Zusammenschaltung von zwei Destillationskolonnen der Sumpfstrom der ersten Kolonne in einem zusätzlichen Verdampfer teilweise oder vollständig verdampft wird und anschließend der zweiten Kolonne zweiphasig oder in Form eines gasförmigen und eines flüssigen Stromes zugeführt wird.

Insbesondere wird der Zulaufstrom (Feed) zur Kolonne (TK oder Destillationskolonne ohne T) teilweise oder vollständig vorverdampft und der Kolonne zweiphasig oder in Form eines gasförmigen und eines flüssigen Stromes zugeführt.

Bevorzugt ist, dass die Trennwand nicht in die Kolonne eingeschweißt ist, sondern in Form von lose gesteckten und adäquat abgedichteten Teilsegmenten gestaltet ist.

Die vorgenannte lose Trennwand besitzt bevorzugt interne Mannlöcher oder herausnehmbare Segmente, die es erlauben, innerhalb der Kolonne von einer Seite der Trennwand auf die andere Seite zu gelangen.

Bevorzugt ist, dass die Flüssigkeitsverteilung in den einzelnen Teilbereichen der Kolonne (TK) gezielt ungleichmäßig eingestellt werden kann.

Bevorzugt wird in den Teilbereichen 2 und 5 die Flüssigkeit verstärkt im Wandbereich aufgegeben und in den Teilbereichen 3 und 4 die Flüssigkeit reduziert im Wandbereich aufgegeben.

Trennwandkolonnen können, wie bereits erwähnt, im erfindungsgemäßen Verfahren auch durch jeweils zwei thermisch gekoppelte Kolonnen ersetzt werden. Dies ist vor allem dann günstig, wenn die Kolonnen schon vorhanden sind oder die Kolonnen bei verschiedenen Drücken betrieben werden sollen. Bei thermisch gekoppelten Kolonnen kann es von Vorteil sein, den Sumpfstrom der ersten Kolonne in einem zusätzlichen Verdampfer teilweise oder vollständig zu verdampfen und danach der zweiten Kolonne zuzuführen. Diese Vorverdampfung bietet sich insbesondere dann an, wenn der Sumpfstrom der ersten Kolonne größere Mengen an Mittelsieder enthält. In diesem Fall kann die Vorverdampfung auf einem niedrigeren Temperaturniveau erfolgen und der Verdampfer der zweiten Kolonne entlastet werden. Weiterhin wird durch diese Maßnahme der Abtriebsteil der zweiten Kolonne wesentlich entlastet. Der vorverdampfte Strom kann dabei der zweiten Kolonne zweiphasig oder in Form von zwei separaten Strömen zugeführt werden.

Darüber hinaus kann es sowohl bei Trennwandkolonnen als auch bei thermisch gekoppelten Kolonnen von Vorteil sein, den Zulaufstrom einer Vorverdampfung zu unterziehen und anschließend zweiphasig oder in Form von zwei Strömen der Kolonne zuzuführen. Diese Vorverdampfung bietet sich besonders dann an, wenn der Zulaufstrom größere Mengen an Leichtsiedern enthält. Durch die Vorverdampfung kann der Abtriebsteil der Kolonne wesentlich entlastet werden.

Trennwandkolonnen und thermisch gekoppelte Kolonnen können sowohl als Packungskolonnen mit Füllkörpern oder geordneten Packungen oder als Bodenkolonnen ausgeführt werden.

Bei der genannten Reindestillation von DETA und Rückgewinnung von MEOA, die bevorzugt im Vakuum betrieben wird, empfiehlt es sich, Packungskolonnen einzusetzen. Dabei sind geordnete Blechpackungen mit einer spezifischen Oberfläche von 100 bis 500 m²/m³, bevorzugt etwa 250 bis 350 m²/m³, besonders geeignet.

Bei der Reindestillation von EDA und PIP, die bevorzugt bei Drücken leicht oberhalb des atmosphärischen Drucks betrieben wird, damit die Temperatur in allen Bereichen der Kolonne über der Schmelztemperatur von PIP liegt, können sowohl Böden als auch Packungen eingesetzt werden. Als Böden eignen sich besonders Ventilböden. Im Falle von Packungen sind geordnete Blechpackungen mit einer spezifischen Oberfläche von 100 bis 500 m²/m³, bevorzugt etwa 250 bis 350 m²/m³, besonders geeignet.

Die Reindestillation von AEEA wird bevorzugt im Vakuum durchgeführt, daher empfiehlt sich auch hier der Einsatz von Packungen als trennwirksame Einbauten. Dabei sind geordnete Blechpackungen mit einer spezifischen Oberfläche von 100 bis 500 m²/m³, bevorzugt etwa 250 bis 350 m²/m³, besonders geeignet.

Bei der Trennung von Mehrstoffgemischen in eine Leichtsieder-, Mittelsieder- und Hochsiederfraktion existieren üblicherweise Spezifikationen über den maximal zulässigen Anteil an Leichtsiedern und Hochsiedern in der Mittelsiederfraktion. Hierbei werden entweder einzelne für das Trennproblem kritische Komponenten, sogenannte Schlüsselkomponenten, oder die Summe von mehreren Schlüsselkomponenten spezifiziert.

Die Einhaltung der Spezifikation für die Hochsieder in der Mittelsiederfraktion wird über das Aufteilungsverhältnis der Flüssigkeit am oberen Ende der Trennwand geregelt. Dabei wird das Aufteilungsverhältnis der Flüssigkeit am oberen Ende der Trennwand so eingestellt, dass die Konzentration der Schlüsselkomponenten für die Hochsiederfraktion in der Flüssigkeit am oberen Ende der Trennwand 10 bis 80 %, bevorzugt 30 bis 50 %, des Wertes ausmacht, der im Seitenabzugsprodukt erzielt werden soll, und die Flüssigkeitsaufteilung dahingehend eingestellt wird, dass bei höheren Gehalten an Schlüsselkomponenten der Hochsiederfraktion mehr und bei niedrigeren Gehalten an Schlüsselkomponenten der Hochsiederfraktion weniger Flüssigkeit auf den Zulaufteil geleitet wird.

Entsprechend wird die Spezifikation für die Leichtsieder in der Mittelsiederfraktion durch die Heizleistung geregelt. Hierbei wird die Heizleistung im Verdampfer so eingestellt, dass die Konzentration an Schlüsselkomponenten der Leichtsiederfraktion in der Flüssigkeit am unteren Ende der Trennwand 10 bis 80 %, bevorzugt 30 bis 50 %, des Wertes ausmacht, der im Seitenabzugsprodukt erzielt werden soll, und die Heizleistung dahingehend eingestellt wird, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Zur Kompensation von Störungen der Zulaufmenge oder der Zulaufkonzentration erwies es sich zudem als vorteilhaft, durch entsprechende Regelvorschriften im Prozessleitsystem sicherzustellen, dass die Mengenströme der Flüssigkeiten, die auf die Kolonnenteile 2 und 5 (vgl. Abbildung 1) aufgegeben werden, nie unter 30 % ihres Normalwertes sinken können.

Zur Entnahme und Aufteilung der Flüssigkeiten am oberen Ende der Trennwand und an der Seitenentnahmestelle eignen sich sowohl innenliegende als auch außerhalb der Kolonne angeordnete Auffangräume für die Flüssigkeit, die die Funktion einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglichen. Bei der Verwendung von gepackten Kolonnen wird die Flüssigkeit zunächst in Sammlern gefasst und von dort aus in einen innenliegenden oder außenliegenden Auffangraum geleitet.

Anstelle einer Trennwandkolonne - die bei einem Neubau hinsichtlich der Investitionskosten zu bevorzugen ist - ist es auch möglich, zwei Destillationskolonnen nach Art einer thermischen Kopplung so zu verschalten, dass sie hinsichtlich des Energiebedarfs einer Trennwandkolonne entsprechen.

Sie können bei Verfügbarkeit von bestehenden Kolonnen eine sinnvolle Alternative zu Trennwandkolonnen sein. Je nach der Trennstufenzahl der vorhandenen Kolonnen können die geeignetsten Formen der Zusammenschaltung ausgewählt werden. Es ist möglich, Schaltungsformen auszuwählen, die es erlauben, dass nur flüssige Verbindungsströme zwischen den einzelnen Destillationskolonnen auftreten. Diese speziellen Verschaltungen bieten den Vorteil, dass die beiden Destillationskolonnen unter verschiedenen Drücken betrieben werden können mit dem Vorteil, dass sie sich besser an die Temperaturniveaus vorhandener Heiz- und Kühlenergien anpassen lassen. Im allgemeinen wird man die Kolonne, an der die Leichtsiederfraktion entnommen wird im Druck um etwa 0,5 bis 1,0 bar höher wählen als die Kolonne, an der die Schwersiederfraktion entnommen wird.

### Beispiel

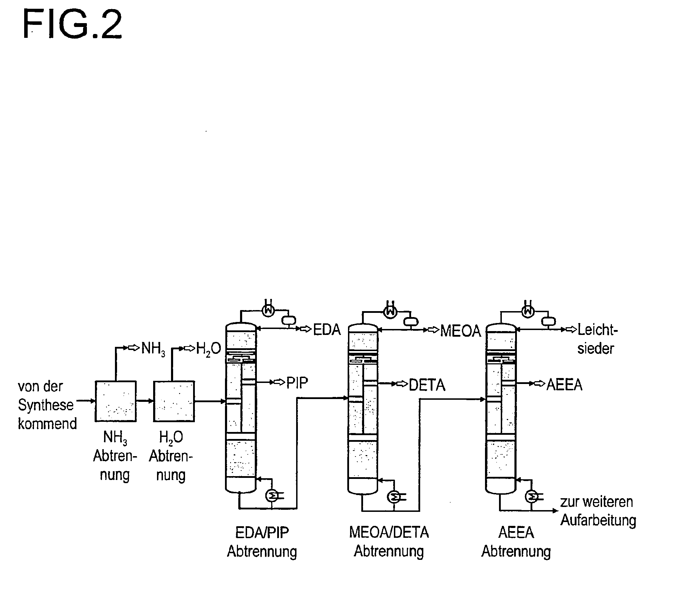

Abbildung 2 zeigt als Beispiel die Auftrennung eines Ethylenamin-Synthesegemisches, nach vorheriger Abtrennung von Ammoniak und Wasser, in Ethylendiamin-Reinprodukt (EDA), Piperazin-Reinprodukt (PIP) und eine Hochsiederfraktion. Die Hochsiederfraktion wird in einer weiteren Trennwandkolonne in Monoethanolamin (MEOA), Diethylentriamin-Reinprodukt (DETA) und eine Hochsiederfraktion aufgetrennt. Last but not least wird aus der am Sumpf der zweiten Trennwandkolonne anfallenden Hochsiederfraktion in einer dritten Trennwandkolonne Aminoethylethanolamin-Reinprodukt (AEEA) und eine weitere Schwersiederfraktion gewonnen. Eventuell vorhandene und im AEEA unerwünschte Leichtsieder werden über Kopf der Kolonne abgezogen.

## Patentansprüche

1. Verfahren zur destillativen Auftrennung von Gemischen enthaltend Ethylenamine, **dadurch gekennzeichnet, dass** es sich bei dem Gemisch enthaltend Ethylenamine um ein Produkt, erhalten durch Umsetzung von Monoethanolamin (MEOA) mit Ammoniak und nachfolgender teilweiser oder vollständiger Abtrennung von Ammoniak und Wasser, handelt, es sich bei den Ethylenaminen um Ethylendiamin (EDA), Piperazin (PIP), Diethylentriamin (DETA), Aminoethylethanolamin (AEEA) und/oder Monoethanolamin (MEOA) handelt und die Auftrennung in einer oder mehreren Trennwandkolonnen durchgeführt wird.

2. Verfahren nach Anspruch 1, **dadurch gekennzeichnet, dass** die Trennwandkolonne (TK) jeweils eine Trennwand (T) in Kolonnenlängsrichtung unter Ausbildung eines oberen gemeinsamen Kolonnenbereichs (1), einen unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (3) und Abtriebsteil (5) aufweist, wobei die Zuführung des aufzutrennenden Gemischs (Feed) im mittleren Bereich des Zulaufteils (2, 4), die Abführung der Hochsiederfraktion über Sumpf (Sumpfabzug C), die Abführung der Leichtsiederfraktion über Kopf (Kopfabzug A) und die Abführung der Mittelsiederfraktion aus dem mittleren Bereich des Entnahmeteils (3, 5) (Seitenabzug B) erfolgt.

3. Verfahren nach einem der vorhergehenden Ansprüche, **dadurch gekennzeichnet, dass** die Trennwandkolonne 30 bis 100 theoretische Trennstufen aufweist bzw. die Trennwandkolonnen jeweils 30 bis 100 theoretische Trennstufen aufweisen.

4. Verfahren nach einem der vorhergehenden Ansprüche, **dadurch gekennzeichnet, dass** das Gemisch enthaltend Ethylenamine in einer Trennwandkolonne aufgearbeitet wird, in der EDA als Kopfprodukt und PIP als Seitenabzugsstrom gewonnen wird, wobei der Betriebsdruck, worunter der am Kopf der Kolonne gemessene absolute Druck zu verstehen ist, 0,1 bis 5 bar beträgt.

5. Verfahren nach einem der vorhergehenden Ansprüche, **dadurch gekennzeichnet, dass** nach der Abtrennung von EDA und PIP in einer Trennwandkolonne weiter aufgearbeitet wird, in der MEOA als Kopfprodukt gewonnen wird und DETA als Seitenabzugsstrom gewonnen wird, wobei der Betriebsdruck, worunter der am Kopf der Kolonne gemessene absolute Druck zu verstehen ist, 0,01 bis 2,5 bar beträgt.

6. Verfahren nach einem der vorhergehenden Ansprüche, **dadurch gekennzeichnet, dass** nach der Abtrennung von EDA, PIP, MEOA und DETA in einer Trennwandkolonne weiter aufgearbeitet wird, in der AEEA als Seitenabzugsstrom gewonnen wird, wobei der Betriebsdruck, worunter der am Kopf der Kolonne gemessene absolute Druck zu verstehen ist, 0,001 bis 1,0 bar beträgt.

7. Verfahren nach dem vorhergehenden Anspruch, **dadurch gekennzeichnet, dass** das Sumpfprodukt der Trennwandkolonne zur Gewinnung von AEEA in einer oder mehreren weiteren konventionellen Destillationskolonnen zur Aufkonzentrierung und Reinigung weiterer, höher siedender Ethylenamine und/oder Ethylenaminoalkohole weiter aufgearbeitet wird.

8. Verfahren nach Anspruch 4, **dadurch gekennzeichnet, dass** der Sumpfstrom der Trennwandkolonne in weiteren konventionellen Destillationskolonnen weiter aufgearbeitet wird, wobei zuerst MEOA in einer Destillationskolonne als Kopfprodukt gewonnen wird und aus dem Sumpfstrom dieser Kolonne in der nächsten Kolonne DETA als Kopfprodukt gewonnen wird, und der Sumpfstrom dieser Kolonne einer oder mehreren weiteren konventionellen Kolonnen zugeführt wird, um AEEA zu gewinnen, oder der Sumpfstrom dieser Kolonne einer Trennwandkolonne zugeführt wird, in der AEEA als Seitenabzugsstrom gewonnen wird.

9. Verfahren nach einem der Ansprüche 1 bis 3, **dadurch gekennzeichnet, dass** das Gemisch enthaltend Ethylenamine einer konventionellen Destillationskolonne zugeführt wird, in der ein EDA/PIP-Gemisch als Kopfprodukt gewonnen wird, und in einer weiteren konventionellen Kolonne in EDA und PIP aufgetrennt wird, und der Sumpfstrom dieser Kolonne in einer Trennwandkolonne weiter aufgearbeitet wird, so dass MEOA als Kopfprodukt gewonnen wird und DETA als Seitenabzugsstrom anfällt, und der Sumpfstrom dieser Trennwandkolonne einer oder mehreren konventionellen Destillationskolonnen zugeführt wird, um AEEA zu gewinnen, oder der Sumpfstrom dieser Trennwandkolonne einer weiteren Trennwandkolonne zugeführt wird, in der AEEA als Seitenabzugsstrom gewonnen wird.

10. Verfahren nach einem der Ansprüche 4 bis 8, **dadurch gekennzeichnet, dass** der obere gemeinsame Kolonnenbereich (1) der Trennwandkolonne (TK) zur Abtrennung von EDA und PIP 5 bis 50 %, der Verstärkungsteil (2) des Zulaufteils (2, 4) der Kolonne 5 bis 50 %, der Abtriebsteil (4) des Zulaufteils der Kolonne 5 bis 50 %, der Verstärkungsteil (3) des Entnahmeteils (3, 5) der Kolonne 5 bis 50 %, der Abtriebsteil (5) des Entnahmeteils der Kolonne 5 bis 50 %, und der gemeinsame untere Bereich (6) der Kolonne 5 bis 50 % der Gesamtzahl der theoretischen Trennstufen der Kolonne aufweist.

11. Verfahren nach einem der Ansprüche 5 bis 7 oder 9, **dadurch gekennzeichnet, dass** der obere gemeinsame Kolonnenbereich (1) der Trennwandkolonne (TK) zur Abtrennung von MEOA und DETA 5 bis 50 %, der Verstärkungsteil (2) des Zulaufteils (2, 4) der Kolonne 5 bis 50 %, der Abtriebsteil (4) des Zulaufteils der Kolonne 5 bis 50 %, der Verstärkungsteil (3) des Entnahmeteils (3, 5) der Kolonne 5 bis 50 %, der Abtriebsteil (5) des Entnahmeteils der Kolonne 5 bis 50 %, und der gemeinsame untere Bereich (6) der Kolonne 5 bis 50 % der Gesamtzahl der theoretischen Trennstufen der Kolonne aufweist.

12. Verfahren nach einem der Ansprüche 6 bis 9, **dadurch gekennzeichnet, dass** der obere gemeinsame Kolonnenbereich (1) der Trennwandkolonne (TK) zur Abtrennung von AEEA 5 bis 50 %, der Verstärkungsteil (2) des Zulaufteils (2, 4) der Kolonne 5 bis 50 %, der Abtriebsteil (4) des Zulaufteils der Kolonne 5 bis 50 %, der Verstärkungsteil (3) des Entnahmeteils (3, 5) der Kolonne 5 bis 50 %, der Abtriebsteil (5) des Entnahmeteils der Kolonne 5 bis 50 %, und der gemeinsame untere Bereich (6) der Kolonne 5 bis 50 % der Gesamtzahl der theoretischen Trennstufen der Kolonne aufweist.

13. Verfahren nach einem der Ansprüche 1 bis 12, **dadurch gekennzeichnet, dass** in der Trennwandkolonne (TK) die Summe der Zahl der theoretischen Trennstufen der Teilbereiche (2) und (4) im Zulaufteil 80 bis 110 % der Summe der Zahl der Trennstufen der Teilbereiche (3) und (5) im Entnahmeteil beträgt.

14. Verfahren nach einem der Ansprüche 4 bis 13, **dadurch gekennzeichnet, dass** die Zulaufstelle und die Seitenabzugsstelle der Trennwandkolonne zur Abtrennung von EDA und PIP hinsichtlich der Lage der theoretischen Trennstufen auf unterschiedlicher Höhe in der Kolonne angeordnet sind, indem sich die Zulaufstelle um 1 bis 10 theoretische Trennstufen von der Seitenabzugsstelle unterscheidet.

15. Verfahren nach einem der Ansprüche 5 bis 14, **dadurch gekennzeichnet, dass** die Zulaufstelle und die Seitenabzugsstelle der Trennwandkolonne zur Abtrennung von MEOA und DETA hinsichtlich der Lage der theoretischen Trennstufen auf unterschiedlicher Höhe in der Kolonne angeordnet sind, indem sich die Zulaufstelle um 1 bis 20 theoretische Trennstufen von der Seitenabzugsstelle unterscheidet.

16. Verfahren nach einem der Ansprüche 6 bis 15, **dadurch gekennzeichnet, dass** die Zulaufstelle und die Seitenabzugsstelle der Trennwandkolonne zur Abtrennung von AEEA hinsichtlich der Lage der theoretischen Trennstufen auf unterschiedlicher Höhe in der Kolonne angeordnet sind, indem sich die Zulaufstelle um 1 bis 20 theoretische Trennstufen von der Seitenabzugsstelle unterscheidet.

17. Verfahren nach einem der Ansprüche 2 bis 16, **dadurch gekennzeichnet, dass** der durch die Trennwand (T) unterteilte Teilbereich der Kolonne (TK) bestehend aus den Teilbereichen 2, 3, 4 und 5 oder Teilen davon mit geordneten Packungen oder Füllkörpern bestückt ist und die Trennwand in diesen Teilbereichen wärmeisolierend ausgeführt ist.

18. Verfahren nach einem der Ansprüche 2 bis 17, **dadurch gekennzeichnet, dass** der durch die Trennwand (T) unterteilte Teilbereich der Kolonne (TK) bestehend aus den Teilbereichen 2, 3, 4 und 5 oder Teilen davon mit Böden bestückt ist und die Trennwand in diesen Teilbereichen wärmeisolierend ausgeführt ist.

19. Verfahren nach einem der Ansprüche 2 bis 18, **dadurch gekennzeichnet, dass** die Mittelsiederfraktion an der Seitenabzugsstelle in flüssiger Form entnommen wird.

20. Verfahren nach einem der Ansprüche 2 bis 18, **dadurch gekennzeichnet, dass** die Mittelsiederfraktion an der Seitenabzugsstelle gasförmig entnommen wird.

21. Verfahren nach einem der Ansprüche 2 bis 20, **dadurch gekennzeichnet, dass** der Brüdenstrom am unteren Ende der Trennwand (T) durch die Wahl und/oder Dimensionierung der Trenneinbauten und/oder den Einbau druckverlusterzeugender Vorrichtungen so eingestellt wird, dass das Verhältnis des Brüdenstroms im Zulaufteil zu dem des Entnahmeteils 0,8 bis 1,2, beträgt.

22. Verfahren nach einem der Ansprüche 2 bis 21, **dadurch gekennzeichnet, dass** die aus dem oberen gemeinsamen Bereich (1) der Kolonne ablaufende Flüssigkeit in einem in der Kolonne oder außerhalb der Kolonne angeordneten Auffangraum gesammelt und gezielt durch eine Festeinstellung oder Regelung am oberen Ende der Trennwand (T) so aufgeteilt wird, dass das Verhältnis des Flüssigkeitsstroms zum Zulaufteil zu dem zum Entnahmeteil 0,1 bis 1,0 beträgt.

23. Verfahren nach einem der Ansprüche 2 bis 22, **dadurch gekennzeichnet, dass** die Flüssigkeit auf den Zulaufteil (Feed) über eine Pumpe gefördert oder über eine statische Zulaufhöhe von mindestens 1 m mengengeregelt aufgegeben wird und die Regelung so eingestellt wird, dass die auf den Zulaufteil aufgegebene Flüssigkeitsmenge nicht unter 30 % des Normalwertes sinken kann.

24. Verfahren nach einem der Ansprüche 2 bis 23, **dadurch gekennzeichnet, dass** die Aufteilung der aus dem Teilbereich 3 im Entnahmeteil der Kolonne ablaufenden Flüssigkeit auf den Seitenabzug und auf den Teilbereich 5 im Entnahmeteil der Kolonne durch eine Regelung so eingestellt wird, dass die auf den Teilbereich 5 aufgegebene Flüssigkeitsmenge nicht unter 30 % des Normalwertes sinken kann.

25. Verfahren nach einem der vorhergehenden Ansprüche, **dadurch gekennzeichnet, dass** die Trennwandkolonne (TK) am oberen und unteren Ende der Trennwand (T) Probenahmemöglichkeiten aufweist und aus der Kolonne kontinuierlich oder in zeitlichen Abständen flüssig oder gasförmig Proben entnommen und hinsichtlich ihrer Zusammensetzung untersucht werden.

26. Verfahren nach einem der vorhergehenden Ansprüche, **dadurch gekennzeichnet, dass** das Aufteilungsverhältnis der Flüssigkeit am oberen Ende der Trennwand (T) so eingestellt wird, dass die Konzentration an denjenigen Komponenten der Hochsiedertraktion, für die im Seitenabzug ein bestimmter Grenzwert für die Konzentration erzielt werden soll, in der Flüssigkeit am oberen Ende der Trennwand 5 bis 75 % des Wertes ausmacht, der im Seitenabzugsprodukt erzielt werden soll, und die Flüssigkeitsaufteilung dahingehend eingestellt wird, dass bei höheren Gehalten an Komponenten der Hochsiederfraktion mehr und bei niedrigeren Gehalten an Komponenten der Hochsiederfraktion weniger Flüssigkeit auf den Zulaufteil geleitet wird.

27. Verfahren nach einem der Ansprüche 2 bis 26, **dadurch gekennzeichnet, dass** die Heizleistung im Verdampfer so eingestellt wird, dass die Konzentration an denjenigen Komponenten der Leichtsiederfraktion, für die im Seitenabzug ein bestimmter Grenzwert für die Konzentration erzielt werden soll, am unteren Ende der Trennwand (T) so eingestellt wird, dass die Konzentration an Komponenten der Leichtsiederfraktion in der Flüssigkeit am unteren Ende der Trennwand 10 bis 99 % des Wertes ausmacht, der im Seitenabzugsprodukt erzielt werden soll, und die Heizleistung dahingehend eingestellt wird, dass bei höherem Gehalt an Komponenten der Leichtsiederfraktion die Heizleistung erhöht und bei niedrigerem Gehalt an Komponenten der Leichtsiederfraktion die Heizleistung verringert wird.

28. Verfahren nach einem der Ansprüche 2 bis 27, **dadurch gekennzeichnet, dass** die Destillatentnahme temperaturgeregelt erfolgt und als Regeltemperatur eine Messstelle im Teilbereich 1 der Kolonne verwendet wird, die um 2 bis 20 theoretische Trennstufen unterhalb des oberen Endes der Kolonne angeordnet ist.

29. Verfahren nach einem der Ansprüche 2 bis 28, **dadurch gekennzeichnet, dass** die Entnahme des Sumpfprodukts temperaturgeregelt erfolgt und als Regeltemperatur eine Messstelle im Teilbereich 6 der Kolonne verwendet wird, die um 2 bis 20, theoretische Trennstufen oberhalb des unteren Endes der Kolonne angeordnet ist.

30. Verfahren nach einem der Ansprüche 2 bis 29, **dadurch gekennzeichnet, dass** die Entnahme des Seitenprodukts im Seitenabzug standgeregelt erfolgt und als Regelgröße der Flüssigkeitsstand im Verdampfer verwendet wird.

31. Verfahren nach einem der vorhergehenden Ansprüche, **dadurch gekennzeichnet, dass** anstelle einer Trennwandkolonne eine Zusammenschaltung von zwei Destillationskolonnen in Form einer thermischen Kopplung verwendet wird.

32. Verfahren nach dem vorhergehenden Anspruch, **dadurch gekennzeichnet, dass** beide thermisch gekoppelten Destillationskolonnen jeweils mit einem eigenen Verdampfer und Kondensator ausgestattet sind.

33. Verfahren nach einem der beiden vorhergehenden Ansprüche, **dadurch gekennzeichnet, dass** die beiden thermisch gekoppelten Kolonnen bei verschiedenen Drücken betrieben werden und in den Verbindungsströmen zwischen den beiden Kolonnen nur Flüssigkeiten gefördert werden.

34. Verfahren nach einem der drei vorhergehenden Ansprüche, **dadurch gekennzeichnet, dass** der Sumpfstrom der ersten Kolonne in einem zusätzlichen Verdampfer teilweise oder vollständig verdampft wird und anschließend der zweiten Kolonne zweiphasig oder in Form eines gasförmigen und eines flüssigen Stromes zugeführt wird.

35. Verfahren nach einem der vorhergehenden Ansprüche, **dadurch gekennzeichnet, dass** der Zulaufstrom zur Kolonne (Feed) teilweise oder vollständig vorverdampft wird und der Kolonne zweiphasig oder in Form eines gasförmigen und eines flüssigen Stromes zugeführt wird.

36. Verfahren nach einem der vorhergehenden Ansprüche, **dadurch gekennzeichnet, dass** die Trennwand nicht in die Kolonne eingeschweißt ist, sondern in Form von lose gesteckten und adäquat abgedichteten Teilsegmenten gestaltet ist.

37. Verfahren nach dem vorhergehenden Anspruch, **dadurch gekennzeichnet, dass** die lose Trennwand interne Mannlöcher oder herausnehmbare Segmente besitzt, die es erlauben, innerhalb der Kolonne von einer Seite der Trennwand auf die andere Seite zu gelangen.

38. Verfahren nach einem der Ansprüche 2 bis 37, **dadurch gekennzeichnet, dass** die Flüssigkeitsverteilung in den einzelnen Teilbereichen der Kolonne (TK) gezielt ungleichmäßig eingestellt werden kann.

39. Verfahren nach dem vorhergehenden Anspruch, **dadurch gekennzeichnet, dass** in den Teilbereichen 2 und 5 die Flüssigkeit verstärkt im Wandbereich aufgegeben wird und in den Teilbereichen 3 und 4 die Flüssigkeit reduziert im Wandbereich aufgegeben wird.

## Claims

1. A process for distillatively separating mixtures comprising ethylenamines, wherein the mixture comprising ethyleneamines is a product obtained by reacting monoethanolamine (MEOA) with ammonia and subsequently partly or fully removing ammonia and water, the ethyleneamines are ethylenediamine (EDA), piperazine (PIP), diethylenetriamine (DETA), aminoethylethanolamine (AEEA) and/or monoethanolamine (MEOA) and the separation is carried out in one or more dividing wall columns.

2. The process according to claim 1, wherein the dividing wall column (DWC) in each case has a dividing wall (DW) in the longitudinal direction of the column to form an upper combined column region (1), a lower combined column region (6), a feed section (2, 4) having rectifying section (2) and stripping section (4), and also a withdrawal section (3, 5) having rectifying section (3) and stripping section (5), and the mixture to be separated (feed) is fed in the middle region of the feed section (2, 4), the high boiler fraction is removed via the bottom (bottom draw C), the low boiler fraction is removed via the top (top draw A) and the medium boiler fraction is removed from the middle region of the withdrawal section (3, 5) (side draw B).

3. The process according to either of the preceding claims, wherein the dividing wall column has from 30 to 100 theoretical plates or the dividing wall columns each have from 30 to 100 theoretical plates.

4. The process according to any of the preceding claims, wherein the mixture comprising ethylenamines is worked up in a dividing wall column in which EDA is obtained as a top product and PIP as a side draw stream at an operating pressure, which is understood to mean the absolute pressure measured at the top of the column, of from 0.1 to 5 bar.

5. The process according to any of the preceding claims, wherein, after the removal of EDA and PIP, further workup is effected in a dividing wall column in which MEOA is obtained as a top product and DETA as a side draw stream at an operating pressure, which is understood to mean the absolute pressure measured at the top of the column, of from 0.01 to 2.5 bar.

6. The process according to any of the preceding claims, wherein, after the removal of EDA, PIP, MEOA and DETA, further workup is effected in a dividing wall column in which AEEA is obtained as a side draw stream at an operating pressure, which is understood to mean the absolute pressure measured at the top of the column, of from 0.001 to 1.0 bar.

7. The process according to the preceding claim, wherein the bottom product of the dividing wall column for obtaining AEEA is worked up further in one or more further conventional distillation columns to concentrate and purify further higher-boiling ethylenamines and/or ethylenamino alcohols.

8. The process according to claim 4, wherein the bottom stream of the dividing wall column is worked up further in further conventional distillation columns to obtain first MEOA as a top product in one distillation column and, from the bottom stream of this column, in the next column, DETA is obtained as a top product, and the bottom stream of this column is fed to one or more further conventional columns in order to obtain AEEA, or the bottom stream of this column is fed to a dividing wall column in which AEEA is obtained as a side draw stream.

9. The process according to any of claims 1 to 3, wherein the mixture comprising ethylenamines is fed to a conventional distillation column in which an EDA/PIP mixture is obtained as a top product, and is separated in a further conventional column into EDA and PIP, and the bottom stream of this column is worked up further in a dividing wall column in such a way that MEOA is obtained as a top product and DETA is obtained as a side draw stream, and the bottom stream of this dividing wall column is fed to one or more conventional distillation columns in order to obtain AEEA, or the bottom stream of this dividing wall column is fed to a further dividing wall column in which AEEA is obtained as a side draw stream.

10. The process according to any of claims 4 to 8, wherein the upper combined column region (1) of the dividing wall column (DWC) for removing EDA and PIP has from 5 to 50%, the rectifying section (2) of the feed section (2, 4) of the column has from 5 to 50%, the stripping section (4) of the feed section of the column has from 5 to 50%, the rectifying section (3) of the withdrawal section (3, 5) of the column has from 5 to 50%, the stripping section (5) of the withdrawal section of the column has from 5 to 50% and the combined lower region (6) of the column has from 5 to 50%, of the total number of theoretical plates of the column.

11. The process according to any of claims 5 to 7 or 9, wherein the upper combined column region (1) of the dividing wall column (DWC) for removing MEOA and DETA has from 5 to 50%, the rectifying section (2) of the feed section (2, 4) of the column has from 5 to 50%, the stripping section (4) of the feed section of the column has from 5 to 50%, the rectifying section (3) of the withdrawal section (3, 5) of the column has from 5 to 50%, the stripping section (5) of the withdrawal section of the column has from 5 to 50% and the combined lower region (6) of the column has from 5 to 50%, of the total number of theoretical plates of the column.

12. The process according to any of claims 6 to 9, wherein the upper combined column region (1) of the dividing wall column (DWC) for removing AEEA has from 5 to 50%, the rectifying section (2) of the feed section (2, 4) of the column has from 5 to 50%, the stripping section (4) of the feed section of the column has from 5 to 50%, the rectifying section (3) of the withdrawal section (3, 5) of the column has from 5 to 50%, the stripping section (5) of the withdrawal section of the column has from 5 to 50% and the combined lower region (6) of the column has from 5 to 50%, of the total number of theoretical plates of the column.

13. The process according to any of claims 1 to 12, wherein the sum of the number of theoretical plates of the subregions (2) and (4) in the feed section in the dividing wall column (DWC) is from 80 to 110% of the sum of the number of plates of the subregions (3) and (5) in the withdrawal section.

14. The process according to any of claims 4 to 13, wherein the feed point and the side draw point of the dividing wall column for removing EDA and PIP are disposed at a different height in the column with regard to the position of the theoretical plates by the feed point differing from the side draw point by from 1 to 10 theoretical plates.

15. The process according to any of claims 5 to 14, wherein the feed point and the side draw point of the dividing wall column for removing MEOA and DETA are disposed at a different height in the column with regard to the position of the theoretical plates by the feed point differing from the side draw point by from 1 to 20 theoretical plates.

16. The process according to any of claims 6 to 15, wherein the feed point and the side draw point of the dividing wall column for removing AEEA are disposed at a different height in the column with regard to the position of the theoretical plates by the feed point differing from the side draw point by from 1 to 20 theoretical plates.

17. The process according to any of claims 2 to 16, wherein the subregion of the column (DWC) which is divided by the dividing wall (DW) and consists of the subregions 2, 3, 4 and 5 or parts thereof is charged with structured packings or random packings and the dividing wall is designed with heat insulation in these subregions.

18. The process according to any of claims 2 to 17, wherein the subregion of the column (DWC) which is divided by the dividing wall (DW) and consists of the subregions 2, 3, 4 and 5 or parts thereof is charged with trays and the dividing wall is designed with heat insulation in these subregions.

19. A process according to any of claims 2 to 18, wherein the medium boiler fraction is withdrawn in liquid form at the side draw point.

20. The process according to any of claims 2 to 18, wherein the medium boiler fraction is withdrawn in gaseous form at the side draw point.

21. The process according to any of claims 2 to 20, wherein the vapor flow rate at the lower end of the dividing wall (DW) is adjusted by the selection and/or dimensioning of the separating internals and/or the installation of pressure drop-inducing apparatus in such a way that the ratio of the vapor flow rate in the feed section to that of the withdrawal section is from 0.8 to 1.2.

22. The process according to any of claims 2 to 21, wherein the liquid descending out of the upper combined region (1) of the column is collected in a collecting chamber disposed in the column or outside the column and is precisely divided by a fixed setting or control at the upper end of the dividing wall (DW) in such a way that the ratio of the liquid flow rate to the feed section to that to the stripping section is from 0.1 to 1.0.

23. The process according to any of claims 2 to 22, wherein the liquid is conveyed to the feed section (feed) via a pump or is introduced with flow control using a static feed head of at least 1 m, and the control is adjusted in such a way that the amount of liquid introduced to the feed section cannot fall below 30% of the normal value.

24. The process according to any of claims 2 to 23, wherein the division of the liquid descending out of the subregion 3 in the withdrawal section of the column to the side draw and to the subregion 5 is adjusted by a control in the withdrawal section of the column in such a way that the amount of liquid introduced to the subregion 5 cannot fall below 30% of the normal value.

25. The process according to any of the preceding claims, wherein the dividing wall column (DWC) has sampling means at the upper and lower end of the dividing wall (DW) and liquid or gaseous samples are taken from the column continuously or at time intervals and investigated with regard to their composition.

26. The process according to any of the preceding claims, wherein the division ratio of the liquid at the upper end of the dividing wall (DW) is adjusted in such a way that the concentration of those components of the high boiler fraction for which a certain limiting value for the concentration is to be achieved in the side draw, in the liquid at the upper end of the dividing wall, is from 5 to 75% of the value which is to be achieved in the side draw product, and the liquid division is adjusted to the effect that more liquid is passed to the feed section at higher contents of components of the high boiler fraction, and less liquid at lower contents of components of the high boiler fraction.

27. The process according to any of claims 2 to 26, wherein the heating output in the evaporator is adjusted in such a way that the concentration of those components of the low boiler fraction for which a certain limiting value for the concentration is to be achieved in the side draw, at the lower end of the dividing wall (DW), is adjusted in such a way that the concentration of components of the low boiler fraction in the liquid at the lower end of the dividing wall is from 10 to 99% of the value which is to be achieved in the side draw product, and the heating output is adjusted to the effect that the heating output is increased at a higher content of components of the low boiler fraction and the heating output is reduced at a lower content of components of the low boiler fraction.

28. The process according to any of claims 2 to 27, wherein the distillate is withdrawn under temperature control and the control temperature used is a measurement point in the subregion 1 of the column which is disposed from 2 to 20 theoretical plates below the upper end of the column.

29. The process according to any of claims 2 to 28, wherein the bottom product is withdrawn under temperature control and the control temperature used is a measurement point in the subregion 6 of the column which is disposed from 2 to 20 theoretical plates above the lower end of the column.

30. The process according to any of claims 2 to 29, wherein the side product in the side draw is withdrawn under level control and the control part used is the liquid level in the evaporator.

31. The process according to any of the preceding claims, wherein, instead of a dividing wall column, a connection of two distillation columns in the form of a thermal coupling is used.

32. The process according to the preceding claim, wherein the two thermally coupled distillation columns are each equipped with a dedicated evaporator and condenser.

33. The process according to either of the two preceding claims, wherein the two thermally coupled columns are operated at different pressures and only liquids are conveyed in the connection streams between the two columns.

34. The process according to any of the three preceding claims, wherein the bottom stream of the first column is partly or fully evaporated in an additional evaporator and subsequently fed to the second column in biphasic form or in the form of a gaseous and of a liquid stream.

35. The process according to any of the preceding claims, wherein the feed stream to the column (feed) is partly or fully preevaporated and is fed to the column in biphasic form or in the form of a gaseous and of a liquid stream.

36. The process according to any of the preceding claims, wherein the dividing wall is not welded into the column, but rather is configured in the form of loosely inserted and adequately sealed sub-segments.

37. The process according to the preceding claim, wherein the loose dividing wall has internal manholes or removable segments which allow access from one side of the dividing wall to the other side within the column.

38. The process according to any of claims 2 to 37, wherein the liquid distribution in the individual subregions of the column (DWC) may be deliberately adjusted in a nonuniform manner.

39. The process according to the preceding claim, wherein the liquid is introduced to an increased extent in the wall region in the subregions 2 and 5 and the liquid is introduced to a reduced extent in the wall region in the subregions 3 and 4.

## Revendications

1. Procédé de séparation distillative de mélanges contenant des éthylène-amines, **caractérisé en ce que** le mélange contenant des éthylène-amines est un produit obtenu par réaction de monoéthanolamine (MEOA) avec de l'ammoniac, puis séparation partielle ou totale d'ammoniac et d'eau, **en ce que** les éthylène-amines sont de l'éthylène-diamine (EDA), de la pipérazine (PIP), de la diéthylène-triamine (DETA), de l'aminoéthyléthanolamine (AEEA) et/ou de la monoéthanolamine (MEOA), et **en ce que** la séparation est réalisée dans une ou plusieurs colonnes à paroi de séparation.

2. Procédé selon la revendication 1, **caractérisé en ce que** la colonne à paroi de séparation (TK) comporte à chaque fois une paroi de séparation (T) dans la direction longitudinale de la colonne, qui forme une zone de colonne commune supérieure (1), une zone de colonne commune inférieure (6), une zone d'alimentation (2, 4) comportant une zone d'enrichissement (2) et une zone de rectification (4), ainsi qu'une zone de soutirage (3, 5) comportant une zone d'enrichissement (3) et une zone de rectification (5), l'introduction du mélange à séparer (Feed) ayant lieu dans la zone intermédiaire de la zone d'alimentation (2, 4), le déchargement de la fraction de point d'ébullition élevé ayant lieu par le fond (sortie de fond C), le déchargement de la fraction de point d'ébullition bas ayant lieu par la tête (sortie de tête A) et le déchargement de la fraction de point d'ébullition moyen ayant lieu à partir de la zone intermédiaire de la partie de soutirage (3, 5) (sortie latérale B).

3. Procédé selon l'une quelconque des revendications précédentes, **caractérisé en ce que** la colonne à paroi de séparation comporte 30 à 100 étapes de séparation théoriques ou les colonnes à paroi de séparation comportent chacune 30 à 100 étapes de séparation théoriques.

4. Procédé selon l'une quelconque des revendications précédentes, **caractérisé en ce que** le mélange contenant les éthylène-amines est traité dans une colonne à paroi de séparation dans laquelle l'EDA est récupérée en tant que produit de tête et la PIP en tant que courant de sortie latérale, la pression d'exploitation, qui se rapporte à la pression absolue mesurée à la tête de la colonne, étant de 0,1 à 5 bar.

5. Procédé selon l'une quelconque des revendications précédentes, **caractérisé en ce qu'**un traitement supplémentaire est réalisé dans une colonne à paroi de séparation après la séparation de l'EDA et de la PIP, dans laquelle la MEOA est récupérée en tant que produit de tête et la DETA en tant que courant de sortie latérale, la pression d'exploitation, qui se rapporte à la pression absolue mesurée à la tête de la colonne, étant de 0,01 à 2,5 bar.

6. Procédé selon l'une quelconque des revendications précédentes, **caractérisé en ce qu'**un traitement supplémentaire est réalisé dans une colonne à paroi de séparation après la séparation de l'EDA, de la PIP, de la MEOA et de la DETA, dans laquelle l'AEEA est récupérée en tant que courant de sortie latérale, la pression d'exploitation, qui se rapporte à la pression absolue mesurée à la tête de la colonne, étant de 0,001 à 1,0 bar.

7. Procédé selon la revendication précédente, **caractérisé en ce que** le produit de fond de la colonne à paroi de séparation pour la récupération de l'AEEA est davantage traité dans une ou plusieurs colonnes de distillation classiques supplémentaires pour la concentration et la purification d'éthylène-amines et/ou éthylène-aminoalcools de point d'ébullition supérieur supplémentaires.

8. Procédé selon la revendication 4, **caractérisé en ce que** le courant de fond de la colonne à paroi de séparation est davantage traité dans des colonnes de distillation classiques supplémentaires, de la MEOA étant tout d'abord récupérée dans une colonne de distillation en tant que produit de tête et de la DETA étant récupérée en tant que produit de tête dans la colonne suivante à partir du courant de fond de cette colonne, et le courant de fond de cette colonne étant introduit dans une ou plusieurs colonnes classiques supplémentaires pour récupérer l'AEEA, ou le courant de fond de cette colonne étant introduit dans une colonne à paroi de séparation pour récupérer l'AEEA en tant que courant de sortie latérale.

9. Procédé selon l'une quelconque des revendications 1 à 3, **caractérisé en ce que** le mélange contenant des éthylène-amines est introduit dans une colonne de distillation classique, dans laquelle un mélange EDA/PIP est récupéré en tant que produit de tête, et séparé en EDA et PIP dans une colonne classique supplémentaire, et le courant de fond de cette colonne est davantage traité dans une colonne à paroi de séparation, de manière à récupérer de la MEOA en tant que produit de tête et à ce que de la DETA se forme en tant que courant de sortie latérale, et le courant de fond de cette colonne à paroi de séparation est introduit dans une ou plusieurs colonnes de distillation classiques pour récupérer l'AEEA, ou le courant de fond de cette colonne à paroi de séparation est introduit dans une colonne à paroi de séparation supplémentaire, dans laquelle l'AEEA est récupérée en tant que courant de sortie latérale.

10. Procédé selon l'une quelconque des revendications 4 à 8, **caractérisé en ce que** la zone de colonne commune supérieure (1) de la colonne à paroi de séparation (TK) pour la séparation de l'EDA et de la PIP comporte 5 à 50 %, la zone d'enrichissement (2) de la zone d'alimentation (2, 4) de la colonne 5 à 50 %, la zone de rectification (4) de la zone d'alimentation de la colonne 5 à 50 %, la zone d'enrichissement (3) de la zone de soutirage (3, 5) de la colonne 5 à 50 %, la zone de rectification (5) de la zone de soutirage de la colonne 5 à 50 %, et la zone commune inférieure (6) de la colonne 5 à 50 % du nombre total d'étapes de séparation théoriques de la colonne.

11. Procédé selon l'une quelconque des revendications 5 à 7 ou 9, **caractérisé en ce que** la zone de colonne commune supérieure (1) de la colonne à paroi de séparation (TK) pour la séparation de la MEOA et de la DETA comporte 5 à 50 %, la zone d'enrichissement (2) de la zone d'alimentation (2, 4) de la colonne 5 à 50 %, la zone de rectification (4) de la zone d'alimentation de la colonne 5 à 50 %, la zone d'enrichissement (3) de la zone de soutirage (3, 5) de la colonne 5 à 50 %, la zone de rectification (5) de la zone de soutirage de la colonne 5 à 50 %, et la zone commune inférieure (6) de la colonne 5 à 50 % du nombre total d'étapes de séparation théoriques de la colonne.

12. Procédé selon l'une quelconque des revendications 6 à 9, **caractérisé en ce que** la zone de colonne commune supérieure (1) de la colonne à paroi de séparation (TK) pour la séparation de l'AEEA comporte 5 à 50 %, la zone d'enrichissement (2) de la zone d'alimentation (2, 4) de la colonne 5 à 50 %, la zone de rectification (4) de la zone d'alimentation de la colonne 5 à 50 %, la zone d'enrichissement (3) de la zone de soutirage (3, 5) de la colonne 5 à 50 %, la zone de rectification (5) de la zone de soutirage de la colonne 5 à 50 %, et la zone commune inférieure (6) de la colonne 5 à 50 % du nombre total d'étapes de séparation théoriques de la colonne.

13. Procédé selon l'une quelconque des revendications 1 à 12, **caractérisé en ce que** dans la colonne à paroi de séparation (TK), la somme du nombre des étapes de séparation théoriques des zones (2) et (4) de la zone d'alimentation est de 80 à 110 % de la somme du nombre des étapes de séparation théoriques des zones (3) et (5) de la zone de soutirage.

14. Procédé selon l'une quelconque des revendications 4 à 13, **caractérisé en ce que** l'emplacement d'alimentation et l'emplacement de sortie latérale de la colonne à paroi de séparation pour la séparation de l'EDA et de la PIP sont placés à différentes hauteurs dans la colonne au regard de la position des étapes de séparation théoriques, l'emplacement d'alimentation différant de 1 à 10 étapes de séparation théoriques de l'emplacement de sortie latérale.

15. Procédé selon l'une quelconque des revendications 5 à 14, **caractérisé en ce que** l'emplacement d'alimentation et l'emplacement de sortie latérale de la colonne à paroi de séparation pour la séparation de la MEOA et de la DETA sont placés à différentes hauteurs dans la colonne au regard de la position des étapes de séparation théoriques, l'emplacement d'alimentation différant de 1 à 20 étapes de séparation théoriques de l'emplacement de sortie latérale.

16. Procédé selon l'une quelconque des revendications 6 à 15, **caractérisé en ce que** l'emplacement d'alimentation et l'emplacement de sortie latérale de la colonne à paroi de séparation pour la séparation de l'AEEA sont placés à différentes hauteurs dans la colonne au regard de la position des étapes de séparation théoriques, l'emplacement d'alimentation différant de 1 à 20 étapes de séparation théoriques de l'emplacement de sortie latérale.

17. Procédé selon l'une quelconque des revendications 2 à 16, **caractérisé en ce que** la zone divisée par la paroi de séparation (T) de la colonne (TK) constituée des zones 2, 3, 4 et 5, ou des parties de celle-ci, est chargée avec des garnissages structurés ou des corps de remplissage, et la paroi de séparation de ces zones est conçue sous une forme calorifuge.

18. Procédé selon l'une quelconque des revendications 2 à 17, **caractérisé en ce que** la zone divisée par la paroi de séparation (T) de la colonne (TK) constituée des zones 2, 3, 4 et 5, ou des parties de celle-ci, est chargée avec des plateaux, et la paroi de séparation de ces zones est conçue sous une forme calorifuge.

19. Procédé selon l'une quelconque des revendications 2 à 18, **caractérisé en ce que** la fraction de point d'ébullition moyen est soutirée sous forme liquide à l'emplacement de sortie latérale.

20. Procédé selon l'une quelconque des revendications 2 à 18, **caractérisé en ce que** la fraction de point d'ébullition moyen est soutirée sous forme gazeuse à l'emplacement de sortie latérale.

21. Procédé selon l'une quelconque des revendications 2 à 20, **caractérisé en ce que** le courant de vapeur à l'extrémité inférieure de la paroi de séparation (T) est ajusté par le choix et/ou le dimensionnement des composants de séparation et/ou l'incorporation de dispositifs générant une perte de charge, de manière à ce que le rapport entre le courant de vapeur dans la zone d'alimentation et celui dans la zone de soutirage soit de 0,8 à 1,2.

22. Procédé selon l'une quelconque des revendications 2 à 21, **caractérisé en ce que** le liquide s'écoulant de la zone commune supérieure (1) de la colonne est recueilli dans une chambre de collecte placée dans la colonne ou à l'extérieur de la colonne et réparti de manière ciblée par un ajustement permanent ou un réglage à l'extrémité supérieure de la paroi de séparation (T) de manière à ce que le rapport entre le courant de liquide dans la zone d'alimentation et celui dans la zone de soutirage soit de 0,1 à 1,0.

23. Procédé selon l'une quelconque des revendications 2 à 22, **caractérisé en ce que** le liquide est transporté dans la zone d'alimentation (Feed) par une pompe ou introduit en quantités réglées par une hauteur d'alimentation statique d'au moins 1 m, et **en ce que** le réglage est ajusté de manière à ce que la quantité de liquide introduite dans la zone d'alimentation ne puisse pas chuter en dessous de 30 % de la valeur normale.

24. Procédé selon l'une quelconque des revendications 2 à 23, **caractérisé en ce que** la répartition du liquide s'écoulant à partir de la zone 3 de la zone de soutirage de la colonne dans la sortie latérale et dans la zone 5 de la zone de soutirage de la colonne est ajustée par un réglage de manière à ce que la quantité de liquide introduite dans la zone 5 ne puisse pas chuter en dessous de 30 % de la valeur normale.

25. Procédé selon l'une quelconque des revendications précédentes, **caractérisé en ce que** la colonne à paroi de séparation (TK) comporte des possibilités de prélèvement d'échantillons à l'extrémité supérieure et inférieure de la paroi de séparation (T) et **en ce que** des échantillons liquides ou gazeux sont prélevés de la colonne en continu ou à intervalles dans le temps et analysés au regard de leur composition.

26. Procédé selon l'une quelconque des revendications précédentes, **caractérisé en ce que** le rapport de répartition du liquide à l'extrémité supérieure de la paroi de séparation (T) est ajusté de manière à ce que la concentration des composants de la fraction de point d'ébullition élevé, pour lesquels une valeur limite déterminée pour la concentration doit être ciblée dans la sortie latérale, dans le liquide à l'extrémité supérieure de la paroi de séparation soit de 5 à 75 % de la valeur qui doit être ciblée dans le produit de sortie latérale, et la répartition du liquide est pour cela ajustée de manière à introduire davantage de liquide dans la partie d'alimentation dans le cas de teneurs supérieures en composants de la fraction de point d'ébullition élevé, et moins de liquide dans le cas de teneurs inférieures en composants de la fraction de point d'ébullition élevé.

27. Procédé selon l'une quelconque des revendications 2 à 26, **caractérisé en ce que** la puissance de chauffage dans l'évaporateur est ajustée de manière à ce que la concentration en composants de la fraction de point d'ébullition bas, pour lesquels une valeur limite déterminée pour la concentration doit être ciblée dans la sortie latérale, à l'extrémité inférieure de la paroi de séparation (T) soit de 10 à 99 % de la valeur qui doit être ciblée dans le produit de sortie latérale, et la puissance de chauffage est pour cela ajustée de manière à augmenter la puissance de chauffage dans le cas de teneurs supérieures en composants de la fraction de point d'ébullition bas, et à réduire la puissance de chauffage dans le cas de teneurs inférieures en composants de la fraction de point d'ébullition bas.

28. Procédé selon l'une quelconque des revendications 2 à 27, **caractérisé en ce que** le soutirage du distillat a lieu à température contrôlée et **en ce qu'**un emplacement de mesure dans la zone 1 de la colonne, situé 2 à 20 étapes de séparation théoriques en dessous de l'extrémité supérieure de la colonne, est utilisé en tant que température contrôlée.

29. Procédé selon l'une quelconque des revendications 2 à 28, **caractérisé en ce que** le soutirage du produit de fond a lieu à température contrôlée et **en ce qu'**un emplacement de mesure dans la zone 6 de la colonne, situé 2 à 20 étapes de séparation théoriques au-dessus de l'extrémité inférieure de la colonne, est utilisé en tant que température contrôlée.

30. Procédé selon l'une quelconque des revendications 2 à 29, **caractérisé en ce que** le soutirage du produit latéral dans la sortie latérale a lieu à niveau contrôlé et **en ce que** le niveau de liquide dans l'évaporateur est utilisé en tant que grandeur contrôlée.

31. Procédé selon l'une quelconque des revendications précédentes, **caractérisé en ce qu'**à la place d'une colonne à paroi de séparation, une interconnexion de deux colonnes de distillation sous la forme d'un couplage thermique est utilisée.

32. Procédé selon la revendication précédente, **caractérisé en ce que** les deux colonnes de distillation couplées thermiquement sont chacune munies d'un évaporateur et d'un condensateur propre.

33. Procédé selon l'une quelconque des deux revendications précédentes, **caractérisé en ce que** les deux colonnes couplées thermiquement sont exploitées à des pressions différentes et **en ce que** seuls des liquides sont transportés dans les courants de connexion entre les deux colonnes.

34. Procédé selon l'une quelconque des trois revendications précédentes, **caractérisé en ce que** le courant de fond de la première colonne est évaporé en partie ou en totalité dans un évaporateur supplémentaire, puis introduit dans la seconde colonne sous forme biphasée ou sous la forme d'un courant gazeux et d'un courant liquide.

35. Procédé selon l'une quelconque des revendications précédentes, **caractérisé en ce que** le courant d'alimentation de la colonne (Feed) est pré-évaporé en partie ou en totalité et introduit dans la colonne sous forme biphasée ou sous la forme d'un courant gazeux et d'un courant liquide.

36. Procédé selon l'une quelconque des revendications précédentes, **caractérisé en ce que** la paroi de séparation n'est pas soudée dans la colonne, mais plutôt conçue sous la forme de segments connectés de manière lâche et étanchéifiés de manière adéquate.

37. Procédé selon la revendication précédente, **caractérisé en ce que** la paroi de séparation lâche comporte des trous d'homme internes ou des segments amovible qui permettent de passer d'un côté à l'autre de la paroi de séparation dans la colonne.

38. Procédé selon l'une quelconque des revendications 2 à 37, **caractérisé en ce que** la répartition du liquide dans les zones individuelles de la colonne (TK) peut être ajustée de manière inhomogène ciblée.

39. Procédé selon la revendication précédente, **caractérisé en ce que** le liquide est introduit de manière renforcée dans la zone de la paroi dans les zones 2 et 5, et **en ce que** le liquide est introduit de manière réduite dans la zone de la paroi dans les zones 3 et 4.
